# FASCICULE DE BREVET EUROPEEN

(11) **EP 3 714 834 B1**
(45) Date de publication et mention de la délivrance du brevet: **13.07.2022**
(21) Numéro de dépôt: 19166219.6
(22) Date de dépôt: 29.03.2019
(51) Int. Cl.: A61C 1/12, A61C 1/14

(54) **DISPOSITIF DE SERRAGE ASSISTÉ POUR PIÈCE À MAIN**
VORRICHTUNG ZUM UNTERSTÜTZTEN SPANNEN FÜR EIN HANDSTÜCK
ASSISTED TIGHTENING DEVICE FOR HANDPIECE

(43) Date de publication de la demande: 30.09.2020
(73) Titulaire: Bien-Air Holding SA, 2504 Bienne (CH)
(72) Inventeur: Bernasconi, Fabio, 2603 Péry (CH); SARCHI, Davide, 8008 Zürich (CH); Juillerat, Sébastien, 2740 Moutier (CH); Guerlesquin, Gaël, 2052 Fontainemelon (CH)
(74) Mandataire: BOVARD AG

(56) Documents cités:
- EP-A1- 0 374 276
- FR-A1- 2 573 303
- JP-A- 2002 095 679
- US-A1- 2009 176 187

## Description

### Domaine technique de l'invention

La présente invention se rapporte au domaine des turbines et des contre-angles, plus particulièrement aux dispositifs de serrage d'un outil de fraisage.

### Etat de la technique

La plupart des pièces-à-main dentaires à haute vitesse du marché, c'est-à-dire les turbines (alimentées en air) et les contre-angles multiplicateurs (alimentés par des moteurs électriques) sont équipés d'un système de serrage de la fraise basé sur l'utilisation d'une pince élastique. Ce système est bien connu depuis très longtemps, notamment de la solution décrite dans le document de brevet EP0273259B1.

Dans des systèmes de serrage de ce type, l'arbre du rotor (sur lequel et à l'extérieur duquel on chasse soit la turbine, dans le cas d'une turbine à air, soit le pignon de transmission, dans le cas d'un contre-angle) entraînant l'outil de fraisage en rotation contient une pince et un poussoir. La pince est généralement chassée et/ou soudée dans l'arbre, et elle possède deux fonctions: celle de retenir axialement la fraise, grâce à des extrémités élastiques flexibles, et celle de guider la fraise radialement, pour éviter que, en présence de la charge radiale appliquée par le dentiste pendant le fraisage, la fraise soit excessivement inclinée et puisse générer des vibrations excessives nuisibles à la précision du travail. Le poussoir est quant à lui partiellement libre dans l'arbre parce qu'il peut glisser axialement dans l'arbre et dans certains cas tourner angulairement dans l'arbre (dans le cas d'un poussoir conique); il coopère avec la pince dans la phase d'ouverture de la pince pour libérer la fraise lorsque le chirurgien-dentiste appuie sur le bouton de desserrage qui recouvre le poussoir. Quand la pince est fermée et retient la fraise, le poussoir peut être en appui simple sur une surface de la pince mais il ne doit jamais exercer une force d'ouverture sur les parties élastiques de la pince.

Afin d'empêcher que, lors du fraisage et à cause d'une charge axiale excessive appliquée par le chirurgien-dentiste sur la fraise, la fraise puisse glisser au-delà des mâchoires de la pince vers le couvercle et coincer le poussoir, rendant ainsi impossible à l'utilisateur de pousser le poussoir à travers la pression appliquée sur le couvercle et ouvrir la pince pour extraire la fraise à la fin du travail, il existe par ailleurs des systèmes de serrage perfectionnés comprenant un composant additionnel auquel on se réfère comme étant un arrêt de serrage. Un tel composant est introduit, de préférence chassé et/ou soudé dans l'arbre du côté du couvercle de la tête de la pièce-à-main, et il permet d'éviter tout blocage axial du poussoir. La présence de l'arrêt de serrage limite ainsi les retours du marché dus à une fraise restée bloquée dans le serrage.

Néanmoins, aucune de ces solutions de l'état de la technique ne permet d'éviter efficacement que le poussoir ne force accidentellement l'ouverture de la pince lors de la rotation et du fraisage, notamment à cause de vibrations ou de chocs lorsque la matière à fraiser est particulièrement dure.

On connaît par ailleurs, par exemple du document de brevet EP0374276, l'usage d'aimants dans des pièces à main dentaires. La solution divulguée dans ce document concerne toutefois uniquement un agencement particulier pour le passage d'un liquide de refroidissement, remplaçant parallèlement un contact par friction.

Il existe donc un besoin pour des solutions exemptes de ces limitations connues.

### Exposé sommaire de l'invention

Un but de la présente invention est ainsi de proposer un nouveau dispositif de serrage qui soit plus efficace et plus fiable.

Un autre objectif de la présente invention est de proposer un dispositif de serrage perfectionné qui reste néanmoins compatible avec des têtes de pièces à main standard, et qui ne rendent pas les opérations de montage du bouton-poussoir utilisé pour le desserrage plus complexe.

Ces buts sont atteints grâce aux caractéristiques de la revendication principale, et en particulier grâce au fait que la pièce à main contient un aimant permanent agencé symétriquement autour de l'axe de rotation et qui exerce une force d'attraction magnétique sur le poussoir en direction du bouton-poussoir.

Un avantage de la solution proposée est qu'elle permet de retenir, grâce à la force magnétique, le poussoir dans une position surélevée par rapport à la pince, ce qui permet d'améliorer la robustesse du serrage, même en cas de chocs importants subis par la fraise pendant le fraisage, qui ne peuvent alors que plus difficilement provoquer le coincement du poussoir dans la pince, et encore moins générer une ouverture du serrage conduisant à l'éjection de la fraise.

Parallèlement, la solution proposée permet d'améliorer la fiabilité du serrage tout en minimisant les composants additionnels à introduire dans la construction de la pièce à main, ni avoir d'influence sur les opérations de montage de cette dernière.

Selon un mode de réalisation préférentiel, l'agencement de l'aimant permanent dans l'écrou de retenue du bouton-poussoir permet par ailleurs de conférer une sécurité additionnelle contre toute ouverture intempestive du serrage en raison d'une pression involontaire exercée sur le bouton-poussoir grâce à la force magnétique qui doit être vaincue en plus de la force de rappel élastique exercée par le ressort.

Selon plusieurs variantes, la géométrie du poussoir peut par ailleurs être adaptée pour maximiser la force d'attraction vis-à-vis de l'aimant, à l'aide notamment de protubérances annulaires périphériques orientées dans le sens vertical ou radial afin de s'en rapprocher selon les besoins, ou encore d'un chapeau pourvu d'un bourrelet annulaire périphérique recouvrant le poussoir.

Selon une autre variante avantageuse, l'aimant est recouvert d'un matériau ferromagnétique à des fins de blindage, permettant ainsi de minimiser les effets du champ magnétique généré par l'aimant vis-à-vis de l'extérieur, d'une part, ainsi que de minimiser les perturbations dues à des champs externes.

### Brève description des dessins

La présente invention sera mieux comprise à la lecture de la description qui va suivre, donnée à titre d'exemple et faite en référence aux dessins dans lesquels:
- la figure 1 est une vue de face de la tête d'une pièce à main pourvue d'un outil de fraisage selon un mode de réalisation préférentiel pour la présente invention;
- la figure 2 est une vue en coupe sagittale partielle, le long du plan de coupe (S-S) indiqué sur la figure 1, en trois dimensions d'une pièce à main utilisant une turbine pourvue d'un système de serrage selon un mode de réalisation préféré pour la présente invention;
- la figure 3 montre une vue en coupe de la pièce à main de la figure 2 dans le plan sagittal (S-S);
- la figure 4 montre un agrandissement du dispositif de serrage matérialisé par la loupe (A) sur la figure 3;
- la figure 5 est une vue en coupe sagittale partielle en trois dimensions d'une pièce à main utilisant une turbine pourvue d'un système de serrage selon un autre mode de réalisation préféré pour la présente invention;
- la figure 6 montre une vue en coupe de la pièce à main de la figure 5 dans le plan sagittal;
- la figure 7 montre un agrandissement du dispositif de serrage matérialisé par la loupe (B) sur la figure 6;
- la figure 8 est une vue en coupe sagittale partielle en trois dimensions d'une pièce à main de type contre-angle utilisant non plus de l'air comprimé mais un arbre d'entraînement moteur, et qui est pourvue d'un système de serrage selon encore un autre mode de réalisation préféré pour la présente invention;
- la figure 9 montre une vue en coupe de la pièce à main de la figure 8 dans le plan sagittal;
- la figure 10 montre un agrandissement du dispositif de serrage matérialisé par la loupe (C) sur la figure 6;
- la figure 11 montre un croquis selon une vue en coupe d'une variante pour la réalisation du poussoir selon l'invention, avec des portions protubérantes en matériau ferromagnétique orientées vers le haut, et les figures 11A et 11B respectivement deux vues de dessus pour des réalisations alternatives d'un tel poussoir;
- la figure 12 montre un croquis selon une vue en coupe d'une autre variante pour la réalisation du poussoir avec des portions protubérantes en matériau ferromagnétique orientées radialement, et les figures 12A et 12B respectivement deux vues de dessus pour des réalisations alternatives d'un tel poussoir;
- la figure 13 montre un croquis selon une vue en coupe d'encore une autre variante pour la réalisation d'un poussoir selon l'invention, recouvert d'un chapeau, et la figure 13A illustre une vue de dessus correspondant à une réalisation possible pour un tel chapeau;
- la figure 14 montre un croquis selon une vue en coupe d'un poussoir et d'un aimant selon un autre mode de réalisation préférentiel, dans lequel le bouton-poussoir est pourvu d'un blindage magnétique.

### Description détaillée de l'invention

Dans le cadre de cette demande, la pièce à main dans laquelle est intégré le mécanisme de serrage selon l'invention peut indifféremment consister en une « turbine », lorsque la fraise est entraînée en rotation sous l'action d'air comprimé injecté par le biais d'un conduit, ou en un « contre-angle» utilisant un arbre d'entraînement actionné par un moteur.

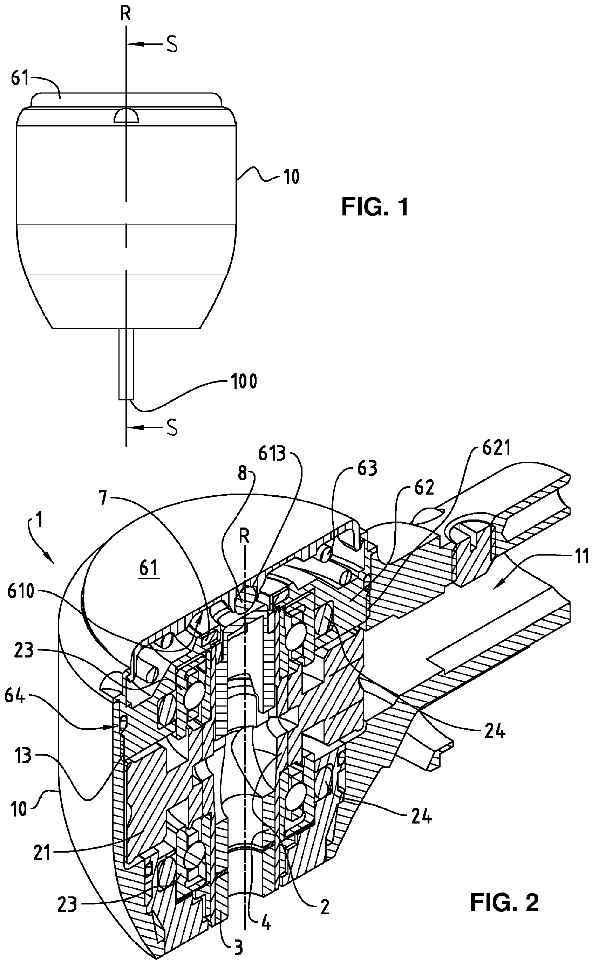

Une vue de l'extérieur d'une pièce à main 1, pouvant consister en une turbine ou un contre-angle, est précisément illustrée sur la figure 1. Elle comprend une tête 10 au bas de laquelle est insérée la fraise 100, montée rotative autour de son axe de rotation R. Un bouton poussoir 61 recouvrant la tête 10 permet classiquement, lorsqu'une pression est exercée sur lui par l'utilisateur de la pièce à main, c'est-à-dire typiquement le chirurgien-dentiste, de libérer la fraise 100.

Les flèches S-S de la figure 1 montrant le plan de coupe sagittal sera utilisé sur les figures 2 à 10 suivantes pour permettre de visualiser plus en détail le mécanisme de montage et de serrage de la fraise 100 proposé dans le cadre de la présente invention, tout d'abord dans le cadre d'une turbine (sur les figures 2 à 7) puis dans le cadre d'un contre-angle.

Dans ce qui suit, on décrira tout d'abord un premier mode de réalisation préférentiel lié à une turbine, en lien avec les figures 2 à 4.

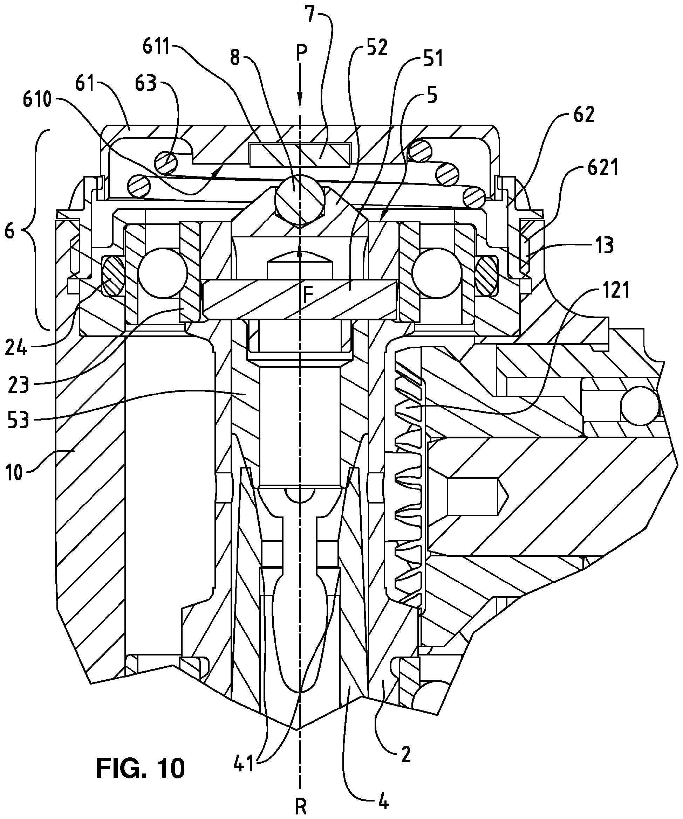

La figure 2, qui consiste en une vue en coupe sagittale partielle en trois dimensions d'une pièce à main 1 le long du plan de coupe (S-S) indiqué sur la figure 1, permet de distinguer les différents éléments usuels d'une tête 10 et d'un mécanisme de serrage pour la fraise 100 - néanmoins non représentée dans un souci de clarté - comprenant notamment le conduit d'approvisionnement en air comprimé 11 sur la droite de la figure, entraînant la turbine 21, elle-même solidaire en rotation d'un arbre d'entraînement 2 pour l'outil de fraisage 100, auquel on se réfère communément comme étant « l'arbre du rotor ». L'arbre d'entraînement 2 est ici inséré entre deux roulements 23, de préférence à billes, eux-mêmes comprimés par des joints de type O-rings 24 respectifs. Dans l'arbre d'entraînement 2 sont montés respectivement la pince 4 de retenue axiale de la fraise - dont les mâchoires ne sont pas visibles sur cette figure, mais mieux visibles sur la figure 10 relative au mode de réalisation correspondant à un contre-angle - et le canon 3 de guidage radial, ces deux pièces étant ici dissociées pour pouvoir notamment ajuster les matériaux utilisés pour la pince 4 de ceux du canon 3 de guidage. En effet, alors que la pince 4 requiert des matériaux de préférence élastiques et déformables, le canon 3 requiert quant à lui de préférence des matériaux très durs, de manière à être inusables et garantir d'excellentes propriétés de guidage le long de l'axe de rotation R le plus longtemps possible.

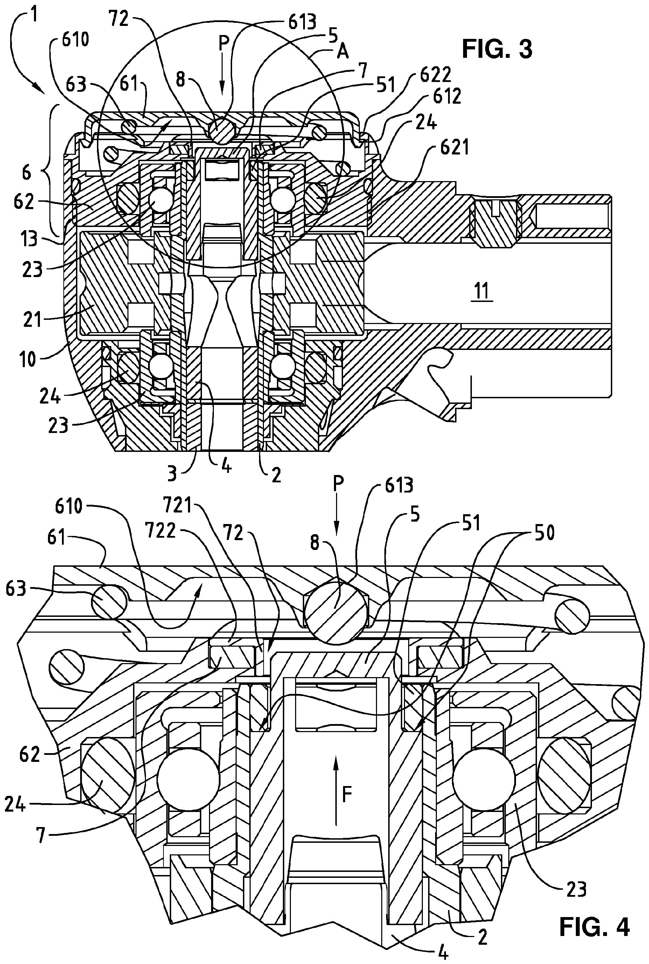

Dans la partie supérieure de la tête est insérée une pièce de retenue pour le bouton-poussoir 61, qui est prévu pour libérer la fraise 100. Cette pièce de retenue consiste, selon le mode de réalisation préférentiel décrit, en un écrou 62 vissé dans la tête par l'intermédiaire d'un bourrelet fileté 621 au niveau de sa périphérie externe, coopérant avec un taraudage 13 pratiqué dans la tête 10; un joint d'étanchéité 64 est prévu au niveau de la coopération de ces surfaces de vissage afin d'assurer le positionnement répétable de l'écrou et du bouton-poussoir et, dans le cas d'une turbine, de stabiliser ainsi les flux aérodynamiques sortant de la tête. Sur le dessus de l'écrou 62 de retenue est disposé un ressort 63 permettant de ramener le bouton-poussoir 61 dans sa position de repos, telle qu'elle est représentée sur les figures 2 et 3, où une partie d'accrochage périphérique radiale 612 du bouton-poussoir est maintenue en appui contre un bec de retenue périphérique intérieur 622, la coopération de parties d'accrochage étant mise en évidence sur la figure 3.

Sur la figure 2, on peut également distinguer, au niveau de face inférieure 610 du bouton-poussoir 61, une bille 8 anti-chauffe, réalisée dans un matériau à faible conductivité thermique prévu à cet effet, et qui est de préférence chassée directement dans un logement central 613 aménagé dans le bouton-poussoir 61, ici de section hexagonale afin de minimiser les ponts thermiques de transfert de chaleur vers le bouton-poussoir 61.

Le module poussoir 6, formé par l'écrou 62 et le bouton-poussoir 61, ainsi que le ressort 63 de compression interposé entre les deux, est illustré plus loin sur la figure 3. Sur la figure 2, on peut néanmoins d'ores et déjà distinguer le poussoir 5, avec lequel le bouton-poussoir 61, ou ici plus exactement la bille 8 peut être amenée à venir en contact, lorsqu'une pression est exercée dans la direction P, visible sur les figures 3 et 4, et un aimant permanent 7 agencé dans l'écrou 62. Dans le cadre de la présente invention, le poussoir 5 est réalisé dans un matériau ferromagnétique passif, de manière à coopérer avec l'aimant permanent 7.

Dans ce qui suit, on se référera indifféremment à la figure 3, reprenant les mêmes éléments que la figure 2 précédemment décrite dans le plan sagittal S-S, et la figure 4, qui montre le détail de la loupe A matérialisée par un cercle sur la figure 3, et qui représente les différents éléments du dispositif de serrage perfectionné selon l'invention selon une vue agrandie. La description des références communes à toutes ces figures ne sera pas systématiquement répétée.

Sur les figures 2 à 4, on peut constater qu'un aimant permanent de forme annulaire 7 est positionné dans l'écrou 62 de retenue du bouton-poussoir 61 à l'aide d'une pièce de retenue 72 possédant une partie cylindrique 721 et une collerette de retenue axiale 722. Cette pièce peut être fixée de n'importe quelle manière à l'écrou 62, par exemple par chassage, soudage, ou encore vissage, et on peut remarquer que le positionnement de l'aimant 7 de forme annulaire dépasse radialement l'enveloppe du poussoir 5, qui possède par ailleurs des épaulements périphériques 50 afin de pouvoir disposer l'arrêt de serrage 51, de forme annulaire, au-dessus des renfoncements radiaux. Cet aimant 7 est aimanté dans la direction axiale et donc exerce en permanence une force d'attraction dirigée substantiellement vers le haut sur l'élément ferromagnétique partenaire, c'est-à-dire le poussoir 5 du dispositif de serrage. La force d'attraction magnétique F est matérialisée par la flèche sur la figure 4 illustrant le fait que le poussoir 5 est maintenu en position haute en direction du bouton-poussoir 61. En conditions d'utilisation, c'est-à-dire pendant la rotation de l'arbre d'entraînement 2 et le fraisage, cette force d'attraction magnétique F permet de plaquer le poussoir 5 contre l'arrêt de serrage 51, et empêche ainsi le coincement du poussoir 5 dans la pince 4 et l'ouverture accidentelle de cette dernière.

L'aimant permanent 7 est, dans le cadre de la présente invention, de préférence de forme annulaire, ou cylindrique, c'est-à-dire présentant une forme de révolution autour de l'axe de rotation R de la fraise. Selon le mode de réalisation illustré par les figures 2 à 4, le fait que l'aimant permanent 7 soit positionné dans l'écrou 62 a pour conséquence de rapprocher au maximum ce dernier de son élément ferromagnétique partenaire, c'est-à-dire le poussoir 5, et ainsi de maximiser l'intensité de la force magnétique F permettant de le maintenir efficacement dans sa position 'haute', c'est-à-dire que le poussoir est maintenu en contact contre l'arrêt de serrage 51.

Ce mode de réalisation préférentiel selon lequel l'aimant permanent 7 est fixe et situé dans l'écrou 62 de retenue du bouton-poussoir 61 présente par ailleurs un avantage technique additionnel de fiabilité. En effet, lors de l'ouverture du serrage, le bouton poussoir 61 est pressé par l'utilisateur selon la direction P, pour enfoncer le poussoir 5 à l'intérieur de la pince 4 et donc ouvrir les manchons élastiques de la pince 4: dans ce cas, l'utilisateur doit vaincre non seulement la force de tenue du ressort 63 comprimé entre l'écrou 62 et le bouton poussoir 61, mais aussi la force magnétique F entre le poussoir 5 et l'aimant permanent 7, ce qui rajoute une sécurité supplémentaire par rapport au risque de pression accidentelle du bouton-poussoir 61 de la part de l'utilisateur.

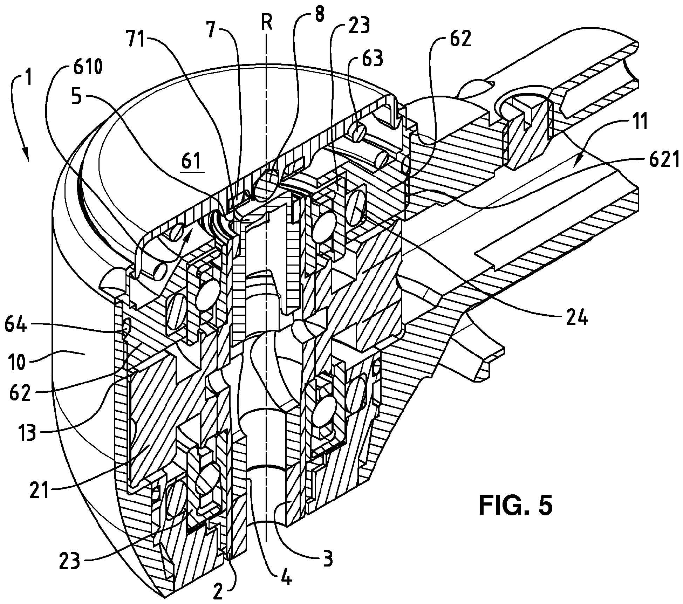

Les figures 5 à 7 illustrent des figures d'un autre mode de réalisation préférentiel pour la présente invention, relatif à une turbine.

Selon ce mode de réalisation, le dispositif d'entraînement de la fraise 100 est en tout point identique à celui du mode de réalisation décrit précédemment et illustré par les figures 2 à 4; par conséquent, toutes les références introduites dans le cadre de ces figures ne seront pas réexpliquées.

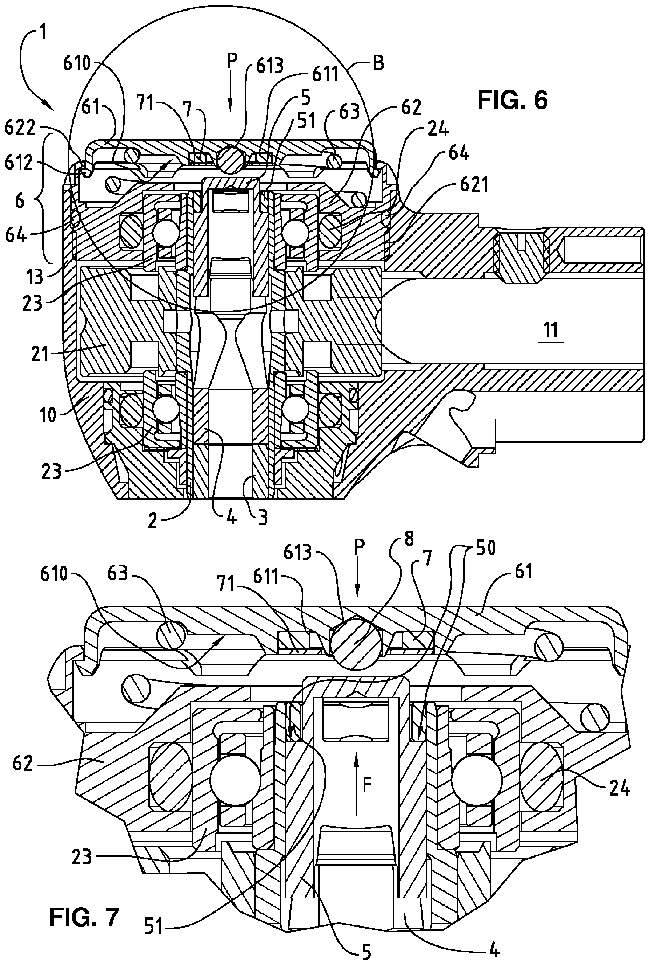

Toutefois, le dispositif de serrage de la fraise 100 présente un agencement différent de l'aimant permanent 7, qui est cette fois non plus intégré dans l'écrou 62, mais directement dans le bouton-poussoir. En effet, comme on peut le constater sur la figure 6, un aimant permanent 7 de forme annulaire est cette fois agencé autour du logement 613 de la bille 8 anti-chauffe, dans un renfoncement 611 dans la face inférieure 610 du bouton-poussoir 61. Il est maintenu en position à l'intérieur du renfoncement du bouton-poussoir 61 par une rondelle de retenue axiale 71, de préférence soudée ou collée au bouton-poussoir 61. L'élément ferromagnétique partenaire de l'aimant est toujours le poussoir 5, représenté sur les figures 5 à 7 dans la position 'haute', c'est-à-dire maintenu en contact contre l'arrêt de serrage 51 grâce à la force d'attraction magnétique F matérialisée par la flèche visible sur la figure 7 et tend à tirer le poussoir 5 vers le haut.

Le mécanisme de fonctionnement est similaire à celui du mode de réalisation précédent en phase de rotation/frasage, où le poussoir est attiré vers le haut suite à la force d'attraction magnétique F exercée par l'aimant. Néanmoins une différence majeure peut être constatée lors de la pression sur le bouton-poussoir 61, illustrée par les flèches P sur les figures 6 et 7. En effet, lors d'une telle phase de pression sur le bouton-poussoir 61, l'attraction magnétique va cette fois-ci favoriser la descente du bouton-poussoir en raison de la distance entre l'aimant permanent 7 qui diminue lorsque le bouton-poussoir se rapproche du poussoir 5. Par conséquent, donc l'intensité de la pression P qui doit être appliquée par l'utilisateur est moindre qu'en absence d'aimant, alors qu'elle était augmentée dans le cas précédent.

Néanmoins, puisque dans le mode de réalisation des figures 5 à 7, l'aimant permanent 7 se trouve dans une position plus éloignée du poussoir 5 que dans le mode de réalisation des figures 2 à 4, la force d'attraction magnétique F exercée sur le poussoir 5 sera inférieure, à égalité de dimensions de l'aimant permanent 7 et du poussoir 5. Ce dernier mode de réalisation des figures 5 à 7 est par contre plus aisé à réaliser, parce qu'une simple rondelle peut être utilisée pour chemiser l'aimant permanent. Du reste, afin d'augmenter la force magnétique exercée sur le poussoir, il serait possible par ailleurs de réaliser la rondelle de retenue axiale 71 également dans un matériau ferromagnétique, de même que la bille 8 anti-chauffe, par exemple en carbure de tungstène avec une teneur de 30 % en Cobalt.

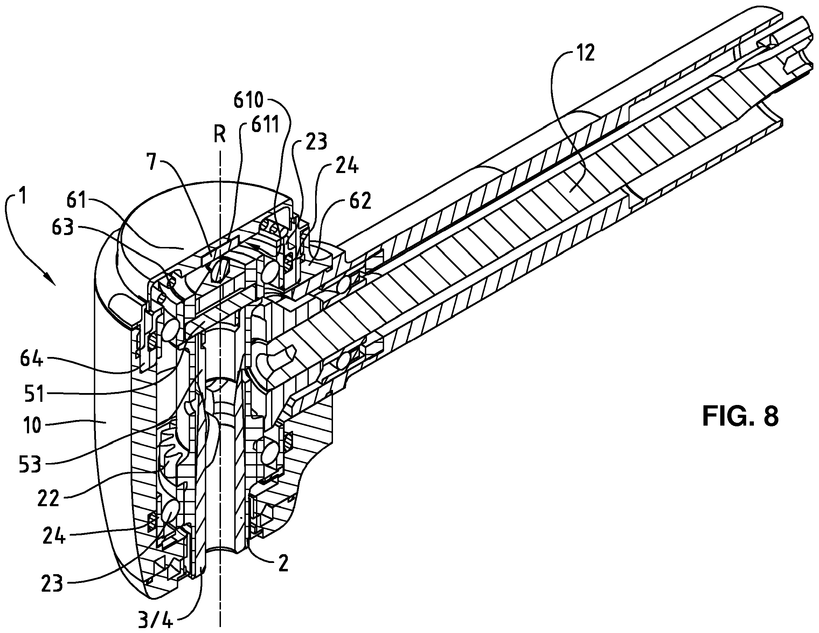

De plus, selon une variante illustrée par les figures 8 à 10 qui suivent, relatif à un contre-angle, l'aimant permanent 7 pourrait aussi prendre la forme d'un disque ou d'une pastille positionnée à l'intérieur du bouton-poussoir, mais derrière la bille 8 anti-chauffe. Dans un tel cas, l'épaisseur de l'aimant permanent 7 pourrait aussi être augmentée de manière considérable pour résulter en une force d'attraction magnétique F accrue.

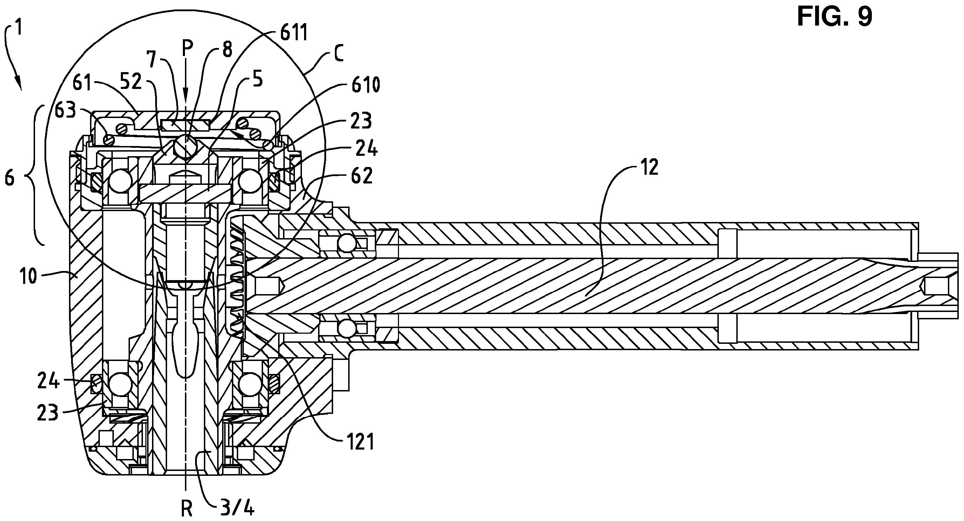

Les figures 8 et 9 mettent en évidence un mode d'entraînement différent de l'arbre du rotor, c'est-à-dire l'arbre d'entraînement 2, par rapport à la turbine des figures 1 à 7 précédentes, cette fois à l'aide d'un engrenage entre l'arbre moteur 12, entraîné par un moteur électrique, et le pignon d'entraînement 22. Le dispositif de serrage ne dissocie plus la pince 4 du canon 3, mais par contre, le poussoir 5 est désormais réalisé en deux parties, avec une partie supérieure 52 du poussoir 5 qui est solidaire en rotation de l'arbre d'entraînement 2, en raison de l'arrêt du poussoir 51 formé par une goupille transversale, et une partie inférieure 53 conique libre en rotation par rapport à l'arbre d'entraînement. L'aimant permanent 7 formé par la pastille disposée dans le renfoncement 611 de la face inférieure 610 du bouton poussoir 61 est désormais susceptible de venir en contact avec la bille 8 anti-chauffe aménagée cette fois non plus directement dans un logement du bouton-poussoir 61, mais sur la partie supérieure 52 du poussoir 5.

Comme on peut le voir sur la figure 10, qui constitue un agrandissement de la partie encerclée - la loupe (C) - de la figure 9, les mâchoires 41 de la pince 4 retiennent axialement la fraise 100 (non représentée pour des questions de lisibilité des autres pièces) tant que la partie inférieure 53 conique ne vient pas les écarter lorsqu'une pression est exercée sur le bouton-poussoir 61 vers l'intérieur de la tête 10, conformément à la direction de la flèche référencée « P ». Dans un tel cas, l'élément magnétique passif associé à l'aimant permanent 7 logé dans le renfoncement 611 aménagé sur la face inférieure du bouton-poussoir 61 consiste précisément au moins en la partie inférieure 53 conique, et, en raison de l'éloignement axial de cette pièce par rapport à l'aimant permanent 7, potentiellement également la partie supérieure 52 du poussoir 5.

Dans les croquis décrits dans ce qui suit, on donnera plusieurs variantes permettant d'augmenter la force d'attraction magnétique F entre l'aimant permanent 7 et le poussoir 5, qui constitue l'élément magnétique passif selon l'invention. On pourra par ailleurs noter que, pour atteindre cet objectif, il serait également possible, selon une variante, de réaliser l'arrêt de serrage également dans un matériau ferromagnétique.

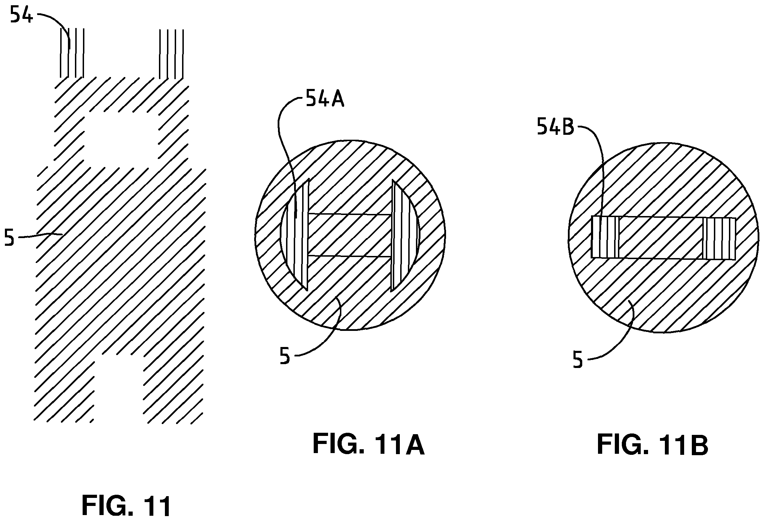

Le croquis de la figure 11 montre une vue en coupe d'une variante pour la réalisation d'un poussoir 5 selon l'invention, avec des premières portions protubérantes annulaires 54 en matériau ferromagnétique orientées vers le haut. Un tel agencement de parties protubérantes formant des sortes de cornes permettent de rapprocher partiellement l'aimant permanent 7 du poussoir 5 et ainsi d'augmenter l'intensité de la force d'attraction magnétique F. Les figures 11A et 11B représentent respectivement deux vues de dessus pour des réalisations alternatives d'un tel poussoir 5 à cornes, avec deux parties périphériques tronquées de disque 54A agencées symétriquement autour de l'axe de rotation R de la fraise, et deux bâtonnets 54B.

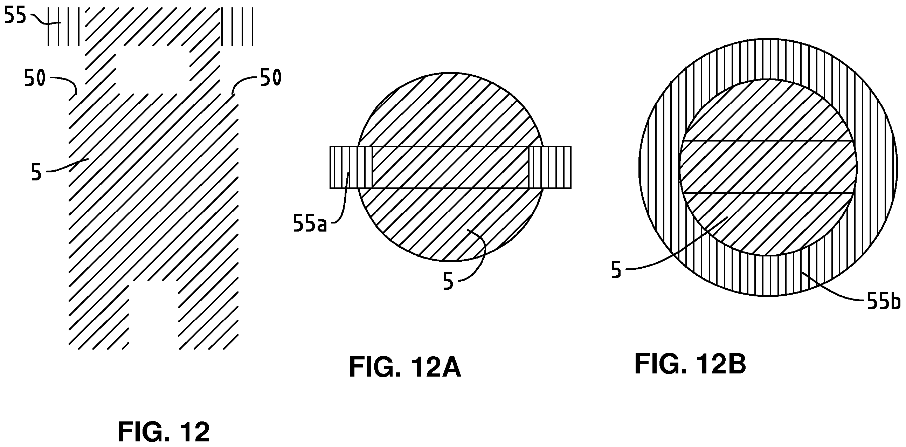

Le croquis de la figure 12 montre une vue en coupe d'une autre variante pour la réalisation du poussoir avec des deuxièmes portions protubérantes 55 en matériau ferromagnétique orientées cette fois radialement, ce qui permet d'obtenir le même effet avantageux d'augmenter l'intensité de la force d'attraction magnétique F en rapprochant au moins partiellement l'aimant permanent 7 du poussoir 5 en le mettant face à face par rapport à l'aimant permanent selon le mode de réalisation illustré sur les figures 2 à 4, ainsi que 5 à 7. Ces deuxièmes portions protubérantes 55 s'étendent radialement au-delà des épaulements 50 du poussoir 5. Les figures 12A et 12B représentent respectivement deux vues de dessus pour des réalisations alternatives de deuxièmes portions protubérantes 55A et 55B, correspondant respectivement encore une fois à une forme de bâtonnets, ou une collerette 55B, pour un tel poussoir 5.

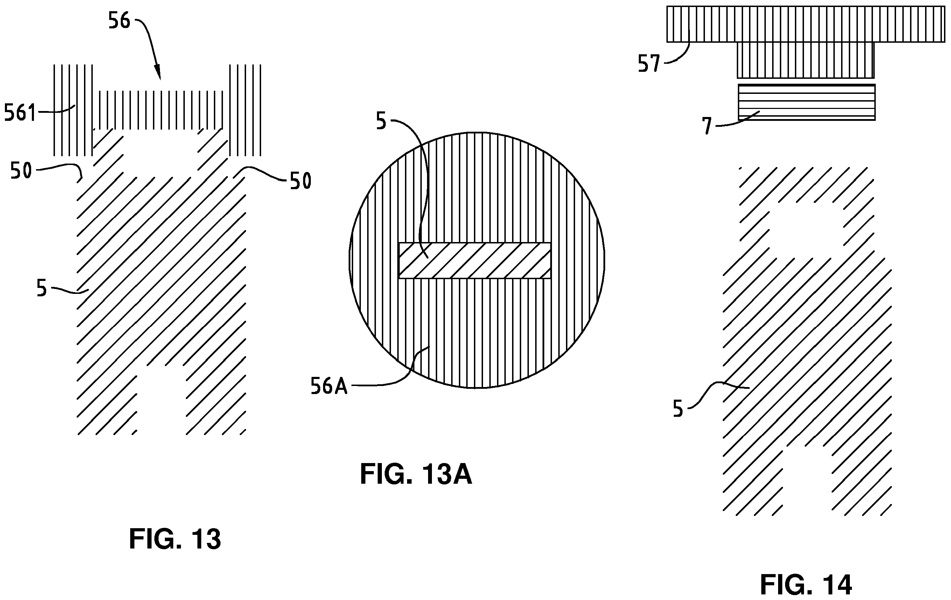

La figure 13 montre un croquis selon une vue en coupe d'encore une autre variante pour la réalisation d'un poussoir 5 selon l'invention dont la forme permet de le rapprocher de l'aimant permanent. Le poussoir 5 est cette fois recouvert d'un chapeau 56, en matériau magnétique, qui est pourvu d'un bourrelet annulaire 561 s'étend à la fois dans le sens axial et dans le sens radial. Une telle variante cumule en quelque sorte les avantages des deux variantes décrites précédemment en relation avec les figures 11 et 12. La figure 13A illustre une vue de dessus correspondant à une réalisation possible pour un tel chapeau en forme de disque surmoulé 56A.

La figure 14 montre enfin quant à elle un croquis selon une vue en coupe d'un poussoir 5 et d'un aimant permanent 7 selon une variante relative au mode de réalisation préférentiel dans lequel l'aimant permanent est intégré au bouton-poussoir 61 - comme illustré notamment sur les figures 5 à 7 et 8 à 10 - dans laquelle le bouton-poussoir 61 est pourvu par ailleurs d'un blindage magnétique. A cet effet, l'aimant permanent 7 est recouvert d'un capot 57 de blindage, réalisé par exemple en Mu-Métal, et qui est intégré audit bouton poussoir 61, soit en constituant le matériau du bouton-poussoir 61 lui-même, soit une armature intérieure.

Bien que l'invention relative au nouveau dispositif de serrage amélioré grâce à l'exercice d'une force magnétique agissant sur le poussoir pour le maintenir dans une position haute empêchant tout desserrement de la pince ait été décrite en lien avec des modes de réalisation distincts illustrés par les ensembles de figures qui précèdent, on comprendra qu'une combinaison notamment des deux modes de réalisation préférentiels relatifs aux turbines serait envisageable en vue de maximiser l'intensité de la force d'attraction magnétique F vers le haut en direction du bouton-poussoir 61. Par ailleurs, on comprendra également que la bille 8 anti-chauffe pourra être réalisée en matériau ferromagnétique pour n'importe lequel des modes de réalisation décrits dans ce qui précède, y compris ceux relatifs au contre-angle illustré sur les figures 8 à 10.

L'homme du métier comprendra par ailleurs qu'il est également envisageable d'agencer le dispositif de serrage et les pièces magnétiques coopérant mutuellement non pas dans la tête 10 de la pièce à main 1 toute entière, mais dans un module ou une cartouche destinée à être installée dans la tête.

Néanmoins, au vu des explications qui précèdent et de l'effet technique recherché, on comprendra qu'il serait désavantageux d'agencer un aimant permanent 7 directement dans le poussoir 5 et d'inverser la disposition des pièces ferromagnétiques dans le module poussoir 6, car la pince 4 étant la plupart du temps réalisée dans un matériau ferromagnétique également, l'attraction magnétique mutuelle entre le poussoir 5 et la pince aurait l'effet inverse de celui recherché.

Pour tous les modes de réalisation illustrés, une variante particulièrement avantageuse consiste par ailleurs à fabriquer le poussoir dans un alliage de fer dont la dureté est inférieure ou égale à 800 HV et revêtu d'une couche mince dure dont la dureté est égale ou supérieure à 900 HV. A titre d'exemple non limitatif, le poussoir peut être fabriqué en acier inoxydable martensitique ou en alliage cobalt-fer. De cette manière, la résistance à la corrosion, la permittivité magnétique et la saturation magnétique du corps du poussoir peuvent être maximisées grâce à l'utilisation du matériau relativement peu dur (typiquement 150 - 700 HV pour les aciers martensitiques trempés ou non trempés, ou les autres alliages fer-cobalt inoxydables et fortement ferromagnétiques comme le Vacoflux). A titre d'exemple non limitatif, la couche dure peut être réalisée en nitrure de chrome ou nitrure de titane ou nitrure de tantale ou oxyde de silicium et être déposée par technologie PVD ou CVD ou ALD. De cette manière, la résistance à l'usure des surfaces de contact poussoir-pince et poussoir-arbre est maximisée, et par conséquent la durée de vie augmentée.

Pour tous les modes de réalisation illustrés, une variante particulièrement avantageuse consiste par ailleurs à fabriquer par usinage, découpe ou impression 3D le poussoir dans un matériaux composite formé d'un composant de faible densité, inférieure ou égale à 5 g/cm³ (à titre d'exemple, le silicium ou le polypropylène ou une autre résine), avec l'inclusion de poudres de matériaux ferromagnétique ayant un champ de saturation magnétique égal ou supérieur à 1 T (à titre d'exemple, le fer, le cobalt ou le nickel). L'avantage de cette variante est que le poussoir peut être à la fois très léger et ressentir une force d'attraction magnétique vers l'aimant très intense, en favorisant le maintien du poussoir en position surélevée par rapport à la pince, même dans le cas de chocs très intenses. Des réalisations possibles de cette variante peuvent être déduites par l'homme du métier en se basant sur les articles suivants :
- Fiske, T.J., Gokturk, H.S. & Kalyon, D.M. Journal of Materials Science (1997) 32: 5551. https://doi.org/10.1023/A:1018620407013, Materials Science and International Team (2008).
- Selected Systems from C-Cr-Fe to Co-Fe-S. Springer. p. 22 (Fig. 2 - Phase diagram of the Fe-Si system). doi:10.1007/978-3-540-74196-1_12. ISBN 978-3-540-74193-0. Retrieved 25 December 2011.

Pour tous les modes de réalisation illustrés, une variante particulièrement avantageuse consiste à introduire dans le bouton-poussoir une insertion, visible à l'extérieur, constituée d'un matériau et/ou un fluide magnétostrictif ou magnéto-chromatique, c'est-à-dire dont la forme et/ou la couleur varie en fonction du champ magnétique extérieur. A titre d'exemple non limitatif, des exemples de ces familles de matériaux se trouvent dans les documents suivants :
- Zhuang, Lin et al., « Hydrophilic Magnetochromatic Nanoparticles with Controllable Sizes and Super-high Magnetization for Visualization of Magnetic Field Intensity », Scientific Reports, 2015/11/23/online, 5, 17063, https://doi.org/10.1038/srep17063, https://www.nature.com/articles/srep17063#supplementary-information
- Diane Talbot, « Smart Materials », booklet produced as the 2003 resource for the Institute of Materials, Minerais and Mining Schools Affiliate Scheme

Avantageusement le matériau et/ou le fluide ainsi inséré dans le bouton-poussoir et visible depuis l'extérieur, peut signaler à l'utilisateur et ou au technicien lors d'une réparation dans le cadre d'un service après-vente (SAV), la dégradation du champ magnétique produit par l'aimant. Cette dégradation, due soit à la dégradation et/ou le vieillissement de l'aimant (soumis à des procédures de stérilisation non conformes au mode d'emploi du produit, à température trop élevée ou sous vide poussé), soit à la pénétration dans la tête de la turbine de matériaux fortement magnétiques pouvant déformer les lignes de champs, soit à un choc violent ayant cassé l'aimant ou le poussoir. Dans ce cas, l'utilisateur pourra renvoyer la pièce-à-main pour réparation avant de causer la dégradation d'autres composants internes, et/ou le technicien SAV pourra intervenir spécifiquement pour nettoyer et/ou réparer les composants magnétiques de la pièce-à-main.

## Revendications

1. Pièce à main (1) pour applications dentaires ou chirurgicales comprenant:
- une tête (10) dans laquelle est inséré de façon amovible un outil de fraisage (100) actionnable en rotation autour d'un axe de rotation (R) par l'intermédiaire d'un mécanisme d'entraînement comportant un arbre d'entraînement (2) rotatif autour dudit axe de rotation (R),
- un dispositif de serrage pour ledit outil de fraisage (100), comprenant une pince (4) de serrage disposée à l'intérieur dudit arbre (2) d'entraînement pour le maintien axial dudit outil de fraisage (100) par rapport audit axe de rotation (R), ladite pince (4) coopérant avec un poussoir (5), et
- un bouton-poussoir (61) étant agencé pour agir sur ledit poussoir (5) afin de libérer ledit outil de fraisage (100);
• la pièce à main (1) contenant un aimant permanent (7) agencé symétriquement autour dudit axe de rotation (R), la pièce à main étant **caractérisée en ce que** ledit aimant permanent (7) exerce une force d'attraction magnétique (F) sur ledit poussoir (5) en direction dudit bouton-poussoir (61).

2. Pièce à main (1) selon la revendication 1, ledit aimant permanent (7) possédant une forme annulaire ou cylindrique.

3. Pièce à main (1) selon l'une des revendications 1 ou 2, ledit aimant permanent (7) étant agencé sur un écrou de retenue (62) dudit bouton-poussoir (61) dans la tête (10).

4. Pièce à main (1) selon la revendication 3, ledit aimant permanent (7) étant fixé audit écrou de retenue (62) dudit bouton-poussoir (61) par le biais d'une pièce de retenue (72) pourvue d'une collerette de retenue axiale (722).

5. Pièce à main (1) selon la revendication 1 ou 2, ledit aimant permanent (7) étant agencé solidaire dudit bouton-poussoir (61).

6. Pièce à main (1) selon la revendication 5, ledit aimant permanent (7) étant disposé entre une rondelle de retenue axiale (71) et la face inférieure (610) dudit bouton poussoir (61).

7. Pièce à main (1) selon la revendication 6, ladite rondelle de retenue axiale (71) étant également réalisée dans un matériau ferromagnétique.

8. Pièce à main (1) selon l'une des revendications 5 à 7, une bille anti-chauffe (8) étant aménagée dans le bouton-poussoir (61), ladite bille anti-chauffe (8) étant également réalisée dans un matériau ferromagnétique.

9. Pièce à main (1) selon la revendication 5 à 7, ledit aimant permanent (7) étant formé par une pastille disposée dans un renfoncement (611) de la face inférieure (610) dudit bouton poussoir (61), susceptible de venir en contact avec une bille anti-chauffe (8) aménagée sur une partie supérieure (52) dudit poussoir (5) solidaire en rotation dudit arbre d'entraînement (2).

10. Pièce à main (1) selon l'une des revendications 5 à 9, ledit aimant (7) étant recouvert d'un capot (57) en Mu-Métal intégré audit bouton poussoir (61).

11. Pièce à main (1) selon l'une des revendications précédentes, ledit poussoir (5) comprenant par ailleurs une première protubérance annulaire périphérique (54) s'étendant vers le haut.

12. Pièce à main (1) selon l'une des revendications précédentes, ledit poussoir (5) comprenant par ailleurs une deuxième protubérance annulaire périphérique (55) s'étendant dans la direction radiale au-dessus de l'épaulement (50).

13. Pièce à main (1) selon l'une des revendications précédentes, ledit poussoir (5) étant recouvert d'un chapeau (56) en matériau magnétique, ledit chapeau (56) étant pourvu d'un bourrelet annulaire (561) s'étendant à la fois dans le sens axial et dans le sens radial.

14. Pièce à main selon l'une des revendications précédentes, ledit poussoir (5) étant fabriqué dans un alliage de fer dont la dureté est inférieure ou égale à 800 HV et revêtu d'une couche mince dure dont la dureté est égale ou supérieure à 900 HV.

15. Pièce à main selon l'une des revendications précédentes, ledit poussoir (5) étant fabriqué par usinage, découpe ou impression 3D dans un matériaux composite formé d'un composant de faible densité, inférieure ou égale à 5 g/cm³, avec l'inclusion de poudres de matériaux ferromagnétique, ayant un champ de saturation magnétique égal ou supérieur à 1 T.

## Patentansprüche

1. Handstück (1) für zahnärztliche oder chirurgische Anwendungen, umfassend:
- einen Kopf (10), in welchen ein Fräswerkzeug (100) lösbar einsetzbar ist, das um eine Rotationsachse (R) mittels eines Antriebsmechanismus drehbar ist, der eine Antriebswelle (2) aufweist, die um die Rotationsachse (R) drehbar ist;
- eine Klemmvorrichtung für das Fräswerkzeug (100) mit einer innerhalb der Antriebswelle (2) angeordneten Klemme (4) zum axialen Halten des Fräswerkzeugs (100) relativ zu der Rotationsachse (R), wobei die Klemme (4) mit einem Drücker (5) zusammenwirkt, und
- einen Druckknopf (61), welcher eingerichtet ist, um mit dem Drücker (5) zusammenzuwirken, um das Fräswerkzeug (100) freizugeben;
• das Handstück (1) umfasst einen Permanentmagneten (7), der symmetrisch um die Rotationsachse (R) angeordnet ist, das Handstück (1) ist **dadurch gekennzeichnet, dass** der Permanentmagnet (7) eine magnetische Anziehungskraft (F) auf den Drücker (5) in der Richtung des Druckknopfes (61) ausübt.

2. Handstück (1) nach Anspruch 1, wobei der Permanentmagnet (7) eine ringförmige oder zylindrische Form hat.

3. Handstück (1) nach Anspruch 1 oder 2, wobei der Permanentmagnet (7) an einer Haltemutter (62) des Druckknopfes (61) im Kopf (10) angeordnet ist.

4. Handstück (1) nach Anspruch 3, wobei der Permanentmagnet (7) an der Haltemutter (62) des Druckknopfs (61) mittels eines Haltemittels (72) befestigt ist, welches mit einem axialen Haltekragen (722) versehen ist.

5. Handstück (1) nach Anspruch 1 oder 2, wobei der Permanentmagnet (7) integral mit dem Druckknopf (61) angeordnet ist.

6. Handstück (1) nach Anspruch 5, wobei der Permanentmagnet (7) zwischen einer axialen Haltescheibe (71) und der Unterseite (610) des Druckknopfs (61) angeordnet ist.

7. Handstück (1) nach Anspruch 6, wobei die axiale Haltescheibe (71) ebenfalls aus einem ferromagnetischen Material gefertigt ist.

8. Handstück (1) nach einem der Ansprüche 5 bis 7, wobei eine Überhitzungskugel (8) in dem Druckknopf (61) vorgesehen ist, wobei die Überhitzungskugel (8) ebenfalls aus einem ferromagnetischen Material gefertigt ist.

9. Handstück (1) nach einem der Ansprüche 5 bis 7, wobei der Permanentmagnet (7) von einem Block gebildet ist, der in einer Aussparung (611) an der Unterseite (610) des Druckknopfs (61) angeordnet ist und mit einer Überhitzungskugel (8) in Kontakt bringbar ist, welche an einem oberen Bereich des Drückers (5) angeordnet und drehfest mit der Antriebswelle (2) verbunden ist.

10. Handstück (1) nach einem der Ansprüche 5 bis 9, wobei der Magnet (7) mit einer Mu-Metallabdeckung (57) überdeckt ist, welche in dem Druckknopf (61) integriert ist.

11. Handstück (1) nach einem der vorhergehenden Ansprüche, wobei der Drücker (5) ferner einen ersten peripheren ringförmigen Vorsprung (54) aufweist, der sich nach oben erstreckt.

12. Handstück (1) nach einem der vorhergehenden Ansprüche, wobei der Drücker (5) ferner einen zweiten peripheren ringförmigen Vorsprung (55) aufweist, welcher sich in radialer Richtung über die Schulter (50) erstreckt.

13. Handstück (1) nach einem der vorhergehenden Ansprüche, wobei der Drücker (5) von einer Kappe (56) aus einem magnetischen Material überdeckt ist, wobei die Kappe (56) mit einem ringförmigen Wulst (561) versehen ist, der sich sowohl axial als auch in radial erstreckt.

14. Handstück nach einem der vorhergehenden Ansprüche, wobei der Drücker (5) aus einer Eisenlegierung mit einer Härte kleiner oder gleich 800 HV hergestellt oder mit einer dünnen harten Schicht überzogen ist, deren Härte gleich oder grösser 900 HV ist.

15. Handstück nach einem der vorhergehenden Ansprüche, wobei der Drücker (5) durch maschinelle Bearbeitung, Schneiden oder 3D-Druck aus einem Verbundmaterial hergestellt ist, das aus einer Komponente mit geringer Dichte von weniger als oder gleich 5 g/cm³ unter Einbeziehen von Pulver aus ferromagnetischem Material mit einem magnetischen Sättigungsfeld gleich oder grösser als 1T gebildet ist.

## Claims

1. Handpiece (1) for dental or surgical applications, comprising:
- a head (10) in which is inserted in removable fashion a drilling tool (100) able to be actuated in rotation about an axis of rotation (R) by means of a drive mechanism comprising a drive shaft (2) rotating about the said axis of rotation (R),
- a clamping device for the said drilling tool (100), comprising a chucking clamp (4) disposed inside said drive shaft (2) for axial retention of the said drilling tool (100) with respect to the said axis of rotation (R), the said clamp (4) co-operating with a plunger (5), and
- a push-button (61) being arranged to act upon the said plunger (5) in order to release the said drilling tool (100);
• wherein the handpiece (1) comprises a permanent magnet (7) arranged symmetrically around the said axis of rotation (R), and wherein the handpiece is **characterized in that** the said permanent magnet (7) is exerting a force of magnetic attraction (F) on the said plunger (5) in the direction of the said push-button (61).

2. Handpiece (1) according to claim 1, the said permanent magnet (7) having an annular or cylindrical shape.

3. Handpiece (1) according to one of the claims 1 or 2, the said permanent magnet (7) being arranged on a retaining nut (62) of the said push-button (61) in the head (10).

4. Handpiece (1) according to claim 3, the said permanent magnet (7) being fixed to the said retaining nut (62) of the said push-button (61) by means of a retaining piece (72) provided with an axial retaining collar (722).

5. Handpiece (1) according to claim 1 or 2, the said permanent magnet (7) being arranged in a way unified with the said push-button (61).

6. Handpiece (1) according to claim 5, the said permanent magnet (7) being disposed between an axial retaining washer (71) and the lower face (610) of the said push-button (61).

7. Handpiece (1) according to claim 6, the said axial retaining washer (71) being likewise made of a ferromagnetic material.

8. Handpiece (1) according to one of the claims 5 to 7, an anti-heating ball (8) being arranged in the push-button (61), the said anti-heating ball (8) being likewise made of a ferromagnetic material.

9. Handpiece (1) according to claim 5 to 7, the said permanent magnet (7) being formed by a pad disposed in a recess (611) of the lower face (610) of the said push-button (61), able to come into contact with an anti-heating ball (8) arranged in an upper part (52) of the said plunger (5) integral in rotation with the said drive shaft (2).

10. Handpiece (1) according to one of the claims 5 to 9, the said magnet (7) being covered by a cover (57) in Mu-metal integrated in said push-button (61).

11. Handpiece (1) according to one of the preceding claims, the said plunger (5) further comprising a first peripheral annular protrusion (54) extending upward.

12. Handpiece (1) according to one of the preceding claims, the said plunger (5) further comprising a second peripheral annular protrusion (55) extending in radial direction beyond the shoulder (50).

13. Handpiece (1) according to one of the preceding claims, the said plunger (5) being covered by a cap (56) in magnetic material, the said cap (56) being provided with an annular bead (561) extending at the same time in axial direction and in radial direction.

14. Handpiece according to one of the preceding claims, the said plunger (5) being made of an iron alloy the hardness of which is less than or equal to 800 HV and covered by a thin hard coating the hardness of which is equal to or greater than 900 HV.

15. Handpiece according to one of the preceding claims, the said plunger (5) being manufactured by machining, cutout or 3D printing in a composite material made up of a component of low density, less than or equal to 5 g/cm³, with the inclusion of powders of ferromagnetic material having a field of magnetic saturation equal to or greater than 1 T.
